(19) Europäisches Patentamt
European Patent Office
Office européen des brevets

(11) Veröffentlichungsnummer: **0 297 447 B1**

# (12) EUROPÄISCHE PATENTSCHRIFT

(45) Veröffentlichungstag der Patentschrift: **16.10.91**

(51) Int. Cl.⁵: **C07C 49/597**, C07C 45/54

(21) Anmeldenummer: **88110067.1**

(22) Anmeldetag: **24.06.88**

## (54) Verfahren zur Herstellung von 2-Cyclopentenonen.

(30) Priorität: **30.06.87 DE 3721497**

(43) Veröffentlichungstag der Anmeldung:
**04.01.89 Patentblatt 89/01**

(45) Bekanntmachung des Hinweises auf die Patenterteilung:
**16.10.91 Patentblatt 91/42**

(84) Benannte Vertragsstaaten:
**BE CH DE FR GB LI**

(56) Entgegenhaltungen:
**EP-A- 0 251 111**
**DE-C- 1 110 642**
**US-A- 3 953 514**

**BEILSTEINS HANDBUCH DER ORGANISCHEN CHEMIE, Band 7, 2. Ergänzungswerk, 4. Auflage, Berlin 1948, SPRINGER VERLAG**

**HOUBEN-WEYL; METHODEN DER ORGANISCHEN CHEMIE, Band VII/2a, 4. Auflage, 1973, Seiten 637-641, Stuttgart, GEORG THIEME VERLAG**

**PATENT ABSTRACTS OF JAPAN, Band 4, Nr. 12(C-71), 29. Januar 1980**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**6900 Heidelberg(DE)**
Erfinder: **Vagt, Uwe , Dr.**
**Paul-Neumann-Strasse 44**
**6720 Speyer(DE)**

**EP 0 297 447 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2-Cyclopentenonen durch Umsetzung von 2- oder 3-Hexen-1,6-disäuren oder deren Estern an festen, oxidischen Katalysatoren.

Die zur Herstellung gesättigter cyclischer Ketone angewandte Methode der Cyclisierung von Dicarbonsäuren oder deren Salzen hat sich offenbar auf die Herstellung von ungesättigten cyclischen Ketonen nicht oder nur mit ungenügenden Ergebnissen übertragen lassen. So wurde bei der thermischen Cyclisierung von 3-Hexen-1,6-disäure (Canad. J. Chem. 41 (1963), S. 2718 - 19) das 2-Cyclopentenon lediglich in 3 %iger Ausbeute erhalten.

Die Synthese des unsubstituierten 2-Cyclopentenons durch Oxidation von Cyclopentadien oder Cyclopenten wird z.B. in Organic Synthesis Coll. Vol. V, Seiten 326 - 328 und 414 - 418 beschrieben. Hier wird Cyclopentadien zunächst mit Peressigsäure in Dichlormethan zu einem Gemisch isomerer Cyclopentendiole umgesetzt, das dann anschließend mit p-Toluolsulfonsäure zu 2-Cyclopentenon dehydratisiert wird. Die Ausbeute über beide Stufen beträgt 35 - 40 % d. Th. bezogen auf eingesetztes Cyclopentadien. Neben der schlechten Ausbeute ist diese Methode dadurch stark eingeschränkt, daß die Verfügbarkeit der Ausgangsprodukte nur für die Synthese des unsubstituierten 2-Cyclopentenons gegeben ist.

Erfolgversprechender können 2-Cyclopentenone durch Umsetzung von ungesättigten Carbonsäuren bzw. deren Estern mit Polyphosphorsäure erhalten werden. In Bull. Soc. Chim. France 1970, S. 2981 - 98 werden mehrere Synthesen für substituierte 2-Cyclopentenone nach dieser Methode beschrieben. Allerdings, und das mindert die Anwendungsbreite erheblich, werden dabei grundsätzlich in 3-Stellung substituierte 2-Cyclopentenone als Reaktionsprodukte genannt. Die Ausbeuten liegen in den meisten Fällen deutlich unter 50 %.

Es war deshalb nach einem wirtschaftlich attraktiven und technisch einfach durchführbaren Verfahren zur Herstellung von 2-Cyclopentenonen zu suchen.

Nach dem neuen Verfahren, das diese Anforderungen erfüllt, stellt man 2-Cyclopentenone der allgemeinen Formel

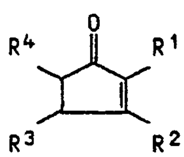

I,

in der $R^1$ bis $R^4$ Wasserstoffatome bedeuten oder für Alkyl- oder Alkenylreste mit 1 bis 12 Kohlenstoffatomen, Cycloalkyl- oder Cycloalkenylreste mit 5 bis 7 Kohlenstoffatomen, Aralkyl- oder Arylreste stehen, dadurch her, daß man Hexendisäuren oder deren Ester der allgemeinen Formel

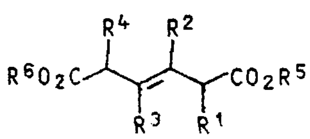

II

oder

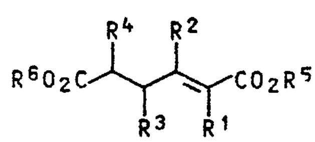

III,

in denen $R^1$ bis $R^4$ die obengenannte Bedeutung haben und $R^5$ und $R^6$ Wasserstoffatome bedeuten oder für Alkylreste mit 1 bis 12 Kohlenstoffatomen, Cycloalkylreste mit 5 oder 6 Kohlenstoffatomen, Aralkyl- oder Arylreste stehen, bei Temperaturen von 150 bis 450 °C an festen, oxidischen Katalysatoren umsetzt.

Die erfindungsgemäße Reaktion läßt sich z.B. für die Umsetzung von 3-Hexen-1,6-disäuredimethylester zu 2-Cyclopentenon durch die folgende Reaktionsgleichung darstellen.:

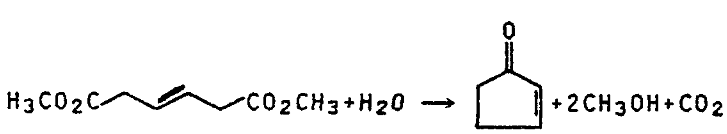

Es war bekannt, 3-Hexen-1,6-disäureester mit Hilfe stöchiometrischer Mengen starker Basen, wie Natriumalkoholaten über das Reaktionszwischenprodukt 2-Cyclopentenon-2-carbonsäureester in zwei Stufen in 2-Cyclopentenon zu überführen. (Diekmann-Kondensation, siehe z.B. JP-OS 77/118 447) Hierbei sind allerdings drei Verfahrensschritte (Kondensation, Neutralisation, Verseifung und Decarboxylierung) notwendig. Darüber hinaus muß die verwendete Base neutralisiert werden, so daß ein erheblicher Zwangsanfall an Neutralsalzen auftritt. Demgegenüber war es überraschend, daß man 2-Cyclopentenone nach dem erfindungsgemäßen Verfahren einstufig und dazu noch mit hoher Ausbeute aus den 2- oder 3-Hexen-1,6-disäuren bzw. deren Estern erhält.

Als Ausgangsstoffe der Formeln II und III kommen 3-Hexen-1,6-disäure oder 2-Hexen-1,6-disäure, die gegebenenfalls durch die Reste $R^1$ bis $R^4$ substituiert sind in Betracht. Die Reste $R^1$ bis $R^4$ können Alkyl- oder Alkenylreste mit 1 bis 12 C-Atomen, wie Methyl-, Ethyl-, Propyl-, Isopropyl-, Butyl-, Isobutyl-, tert.-Butyl-, Hexyl-, Nonyl-, Allyl-, Hexenyl- oder Nonenylreste, Cycloalkyl- oder Cycloalkenylreste mit 5 bis 7 C-Atomen, wie Cyclohexyl-, Cyclopentyl-, 2-Cyclohexenyl-, oder 2-Cyclopentenylreste, Aralkyl- oder Arylreste, wie Phenyl- oder Benzylreste sein. Die Ester der Formeln II unde III sind aliphatische, cycloaliphatische, araliphatische oder aromatische Mono- oder Diester der genannten Dicarbonsäuren. Als Reste $R^5$ und $R^6$ seien z.B. Methyl-, Ethyl-, Propyl-, Isopropyl-, Butyl-, Isobutyl-, tert.-Butyl-, Hexyl-, Nonyl-, Dodecyl-, Cyclopentyl-, Cyclohexyl-, Phenyl- oder Benzylreste genannt.

Beispielsweise können folgende Ausgangsstoffe verwendet werden:

3-Hexen-1,6-disäure, 2-Hexen-1,6-disäure, 2-Methyl-3-hexen-1,6-disäure, 2,5-Dimethyl-3-hexen-1,6-disäure, 3,4-Dimethyl-3-hexen-1,6-disäure, 2-Allyl-3-hexen-1,6-disäure, 3-Cyclohexyl-2-hexen-1,6-disäure, 3-(2-Cyclopentyl)-3-hexen-1,6-disäure, 3-Phenyl-3-hexen-1,6-disäure und 2-Benzyl-3-hexen-1,6-disäure, 3-Hexen-1,6-disäuredimethylester, 2-Hexen-1,6-disäuredimethylester, 3-Hexen-1,6-disäuremonomethylester, 3-Hexen-1,6-disäurediethylester, 2-Hexen-1,6-disäuredibutylester, 3-Hexen-1,6-disäuredicyclohexylester, 3-Hexen-1,6-disäuredibenzylester,2-Methyl-3-hexen-1,6-disäuredimethylester, 2,5-Dimethyl-3-hexen-1,6-disäuredimethylester, 3,4-Dimethyl-3-hexen-1,6-disäuredimethylester, 2-Allyl-3-hexen-1,6-disäuredimethylester, 3-Cyclohexyl-2-hexen-1,6-disäurediethylester, 3-(2-Cyclopentenyl)-3-hexen-1,6-disäuredimethylester, 3-Phenyl-3-hexen-1,6-disäurediethylester oder 2-Benzyl-3-hexen-1,6-disäuredimethylester. Die Umsetzung der Ester ist von besonderem technischen Interesse.

Als Katalysatoren verwendet man feste, oxidische Katalysatoren. Das sind z.B. Oxide von Elementen der I. bis V. Hauptgruppe, der I. bis VIII. Nebengruppe des periodischen Systems der Elemente oder Oxide der Seltenen Erdmetalle oder Gemische der genannten Oxide. So sind beispielsweise Erdalkalioxide, wie Magnesiumoxid, Calciumoxid, Bariumoxid, weiterhin Bortrioxid, Aluminiumoxid, Siliciumoxid, z.B. in Form von Kieselgel, Kieselgur oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide geeignet. Die Katalysatoren können noch durch Aufbringen von Zusätzen, wie Säuren (z.B. Phosphorsäure) oder Basen (z.B. Natriumhydroxid) modifiziert werden. Bevorzugt sind Magnesiumoxid, Bortrioxid, Aluminiumoxid, Siliciumdioxid, Zinkoxid, Titandioxid oder deren Gemische, von denen Aluminiumoxid-Katalysatoren ganz besonders gut geeignet sind.

Es ist zwar möglich, die erfindungsgemäße Umsetzung ohne Zusatz von Wasser durchzuführen, allerdings wird durch die Zugabe von Wasser eine bemerkenswerte Erhöhung von Selektivität und Standzeit erreicht. Das Molverhältnis von Ausgangsstoff II oder III zu Wasser beträgt hierbei vorteilhaft 1:0.05 bis 1:20 insbesondere 1:0.5 bis 1:10.

Die Umsetzung kann in der Gasphase oder in flüssiger Phase, gegebenenfalls auch unter Mitverwendung von Verdünnungsmitteln durchgeführt werden. Als Verdünnungsmittel sind z.B. Lösungsmittel geeignet, die unter den Reaktionsbedingungen vollständig oder weitgehend inert sind, z.B. Ether wie Dioxan oder Tetrahydrofuran. Vorzugsweise wird in der Gasphase gearbeitet, sofern gut verdampfbare Ausgangsstoffe vorliegen.

Die Umsetzung kann diskontinuierlich oder kontinuierlich als Festbettreaktion mit Festbettkatalysatoren, beispielsweise in Sumpf- oder Rieselfahrweise in der Flüssig- oder Gasphase oder als Wirbelbettreaktion mit in auf- und abwirbelnder Bewegung befindlichen Katalysatoren in der Gasphase oder aber mit in der Flüssigphase suspendierten Festbett-Katalysatoren durchgeführt werden.

Die Umsetzung findet bei Temperaturen von 150 bis 450 °C, vorzugsweise bei 200 bis 400 °C, insbesondere 300 bis 345 °C statt. Im allgemeinen wird die Reaktion unter Atmosphärendruck durchgeführt. Es ist jedoch auch möglich, schwach verminderten oder schwach erhöhten Druck, z.B. bis zu 20 bar anzuwenden. Die Katalysatorenbelastung liegt im allgemeinen bei 0.01 bis 40, vorzugsweise 0.1 bis 20 g Ausgangsstoff der Formel II oder III je Gramm Katalysator und Stunde.

Die Umsetzung in der Flüssigphase wird beispielsweise so durchgeführt, daß man ein Gemisch aus der

Ausgangsverbindung und gegebenenfalls Wasser in Gegenwart eines suspendierten Festbettkatalysators auf die gewünschte Reaktionstemperatur erhitzt. Nach Ablauf der notwendigen Reaktionszeit wird das Reaktionsgemisch abgekühlt und der Katalysator, z.B. durch Filtration, entfernt. Das Reaktionsgemisch wird anschließend zur Gewinnung des Ketons bzw. des unumgesetzten Ausgangsstoffes fraktionierend destilliert.

Eine bevorzugte Ausführungsform des erfindungsgemäßen Verfahrens in der Gasphase sieht z.B. so aus, daß man ein Gemisch aus dem Ausgangsstoff der Formel II oder III und Wasser zunächst verdampft und dann, gegebenenfalls zusammen mit einem Inertgas, wie Stickstoff, Kohlendioxid oder Argon, bei der gewünschten Reaktionstemperatur gasförmig in eine in auf- und abwirbelnder Bewegung befindliche Katalysatorschicht einleitet. Der Reaktionsaustrag wird mittels geeigneter Kühlvorrichtungen kondensiert und anschließend durch fraktionierende Destillation aufgearbeitet. Unumgesetzter Ausgangsstoff kann zurückgeführt werden.

Das erfindungsgemäße Verfahren zur Herstellung von 2-Cyclopentenonen besitzt gegenüber den bekannten Verfahren den Vorteil, daß man das Produkt in einem Reaktionsschritt aus den einfach zugänglichen Estern mit hoher Ausbeute und Selektivität erhält.

Die für das erfindungsgemäße Verfahren benötigten Ausgangsstoffe sind durch Metathese von Alkencarbonsäureestern (siehe z.B. J. of Molecular Catalysis 8 (1980), S. 107 - 117) oder durch katalytische Dimerisierung von Acrylestern (z.B. mit Pd-Katalysatoren, siehe J. Org. Chem. 48 (1983), S. 5364 - 66) nicht nur leicht und in guten Ausbeuten, sondern auch mit einem in weiten Grenzen variierbaren Substitutionsmuster herstellbar.

Die nach dem erfindungsgemäßen Verfahren erhältlichen 2-Cyclopentenone sind wertvolle Zwischenprodukte. Das $\alpha,\beta$-ungesättigte Ketonsystem in den 2-Cyclopentenonen ermöglicht eine Vielzahl von Additionsreaktionen vom Michael- oder Diels-Adler-Typ. 2-Cyclopentenone stellen somit wertvolle und vielseitig verwendbare Ausgangsverbindungen für die Synthese von fünfgliedrigen Ringen dar.

Beispiel 1

Pro Stunde wurden 50 ml 3-Hexen-1,6-disäuredimethylester und 33 ml Wasser bei ca. 300 °C verdampft und mit 100 l Stickstoff bei 345 °C über 290 g (350 ml) $\gamma$-Aluminiumoxid-Wirbelkontakt (Korngröße 0.1 - 0.3 mm) geleitet. Der gasförmige Reaktionsaustrag wurde über eine Versuchsdauer von 6 h in Kühlfallen kondensiert und anschließend fraktionierend destilliert. Dabei wurden 74 g (51 % d. Th.) 2-Cyclopentenon vom Siedepunkt 85 - 86 °C/20 mbar und 69 g (22 % d. Th.) unumgesetzter Hexendisäuredimethylester erhalten. Die Ausbeute, bezogen auf umgesetzten Hexendiester, betrug 65 Mol-%.

Beispiel 2

Pro Stunde wurden 50 ml 2-Hexen-1,6-disäuredimethylester und 33 ml Wasser bei ca. 300 °C verdampft und bei 345 °C mit 75 l Stickstoff über 210 g (250 ml) $\gamma$-Aluminiumoxid Wirkelkontakt (Korngröße 0.1 - 0.3 mm) geleitet. Der gasförmige Reaktionsaustrag wurde über eine Versuchsdauer von 6 h in Kühlfallen kondensiert und anschließend fraktionierend destilliert. Dabei wurden 44 g (30 % d. Th.) 2-Cyclopentenon vom Siedepunkt 85 - 86 °C/20 mbar und 91 g (29 % d. Th.) unumgesetzter Hexendisäuredimethylester erhalten. Die Ausbeute, bezogen auf umgesetzten Hexendiester, betrug 42 Mol-%.

Beispiel 3

Pro Stunde wurden 200 ml 3-Hexen-1,6-disäuredimethylester und 33 ml Wasser bei ca. 300 °C verdampft und mit 100 l Stickstoff bei 345 °C über 320 g (350 ml) mit 10 % Bariumoxid dotierten $\gamma$-Aluminiumoxid-Wirbelkontakt (Korngröße 0.1 - 0.3 mm) geleitet. Der gasförmige Reaktionsaustrag wurde über eine Versuchsdauer von 6 h in Kühlfallen kondensiert und anschließend fraktionierend destilliert. Dabei wurden 209 g (36 % d. Th.) 2-Cyclopentenon vom Siedepunkt 85 - 86 °C/20 mbar und 537 g (43 % d. Th.) unumgesetzter Hexendisäuredimethylester erhalten. Die Ausbeute, bezogen auf umgesetzten Hexendiester, betrug 63 Mol-%.

Beispiel 4

Pro Stunde wurden 50 ml 3-Hexen-1,6-disäuredimethylester und 33 ml Wasser bei ca. 300 °C verdampft und bei 345 °C mit 75 l Stickstoff über 320 g (350 ml) mit 10 % Bariumoxid dotierten $\gamma$-Aluminiumoxid-Wirbelkontakt (Korngröße 0.1 - 0.3 mm) geleitet. Der gasförmige Reaktionsaustrag wurde über eine Versuchsdauer von 6 h in Kühlfallen kondensiert und anschließend fraktionierend destilliert. Dabei

wurden 80 g (55 % d. Th.) 2-Cyclopentenon vom Siedepunkt 85 - 86° C/20 mbar und 62 g (20 % d. Th.) unumgesetzter Hexendisäuredimethylester erhalten. Die Ausbeute, bezogen auf umgesetzten Hexendiester, betrug 69 Mol-%.

## Patentansprüche

1. Verfahren zur Herstellung von 2-Cyclopentenonen der allgemeinen Formel

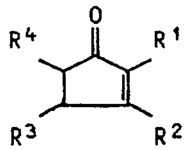

I,

in der $R^1$ bis $R^4$ Wasserstoffatome bedeuten oder für Alkyl- oder Alkenylreste mit 1 bis 12 Kohlenstoffatomen, Cycloalkyl- oder Cycloalkenylreste mit 5 bis 7 Kohlenstoffatomen, Aralkyl- oder Arylreste stehen, dadurch gekennzeichnet, daß man Hexendisäuren oder deren Ester der allgemeinen Formel

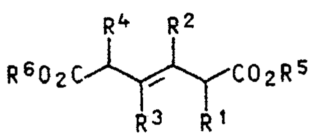

II

oder

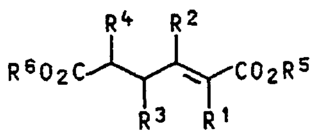

III,

in denen $R^1$ bis $R^4$ die obengenannte Bedeutung haben und $R^5$ und $R^6$ Wasserstoffatome bedeuten oder für Alkylreste mit 1 bis 12 Kohlenstoffatomen, Cycloalkylreste mit 5 oder 6 Kohlenstoffatomen, Aralkyl- oder Arylreste stehen, bei Temperaturen von 150 bis 450° C an festen, oxidischen Katalysatoren umsetzt.

2. Verfahren nach Anspruch 1, dadurch gekennzeichnet, daß man als Katalysatoren Oxide von Elementen der I. bis V. Hauptgruppe, der I. bis VIII. Nebengruppe des Periodischen Systems der Elemente oder Oxide der Seltenen Erdmetalle oder deren Gemische verwendet.

3. Verfahren nach Anspruch 1, dadurch gekennzeichnet, daß man als Katalysator Aluminiumoxid verwendet.

4. Verfahren nach den Ansprüchen 1 bis 3, dadurch gekennzeichnet, daß man die Umsetzung der Ausgangsstoffe der Formel II oder III unter Zusatz von Wasser durchführt, wobei ein Molverhältnis zwischen dem Ausgangsstoff der Formel II oder III und Wasser von 1:0.05 bis 1:20, gewählt wird.

5. Verfahren nach den Ansprüchen 1 bis 4, dadurch gekennzeichnet, daß man die Umsetzung im Wirbelbett durchführt.

6. Verfahren nach den Ansprüchen 1 bis 5, dadurch gekennzeichnet, daß man die Umsetzung bei Temperaturen von 300 bis 345° C vornimmt.

## Claims

1. A process for the preparation of a 2-cyclopentenone of the formula

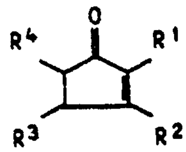

I

where $R^1$ to $R^4$ are each hydrogen, alkyl or alkenyl, each of 1 to 12 carbon atoms, cycloalkyl or cycloalkenyl, each of 5 to 7 carbon atoms, aralkyl or aryl, wherein a hexenedioic acid or an ester thereof of the formula

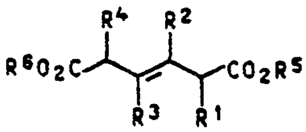

II

or

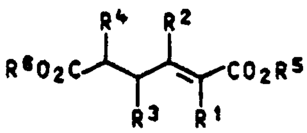

III,

where $R^1$ to $R^4$ have the abovementioned meanings and $R^5$ and $R^6$ are each hydrogen, alkyl of 1 to 12 carbon atoms, cycloalkyl of 5 or 6 carbon atoms, aralkyl or aryl, is converted at from 150 to 450 °C over a solid oxide catalyst.

2. A process as claimed in claim 1, wherein the catalyst used is an oxide of an element of main groups I to V or of subgroups I to VIII of the Periodic Table of Elements or a rare earth metal oxide or a mixture thereof.

3. A process as claimed in claim 1, wherein the catalyst used is alumina.

4. A process as claimed in claim 1 or 2 or 3, wherein the conversion of the starting material of the formula II or III is carried out with the addition of water, the molar ratio of starting material of the formula II or III to water being chosen as from 1 : 0.05 to 1 : 20.

5. A process as claimed in claim 1 or 2 or 3 or 4, wherein the conversion is carried out in a fluidized bed.

6. A process as claimed in claim 1 or 2 or 3 or 4 or 5, wherein the conversion is carried out at from 300 to 345 °C.

## Revendications

1. Procédé de préparation de 2-cyclopenténones de formule générale

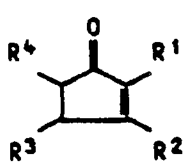

I.

dans laquelle $R^1$ à $R^4$ représentent des atomes d'hydrogène ou sont mis pour des restes alkyle ou alcényle à 1-12 atomes de carbone, pour des restes cycloalkyle ou cycloalcényle à 5-7 atomes de carbone ou pour des restes aralkyle ou aryle, caractérisé en ce qu'on transforme des acides hexénedioïques ou leurs esters de formule générale

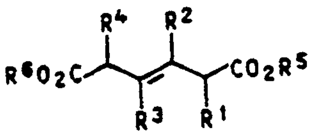

II

ou

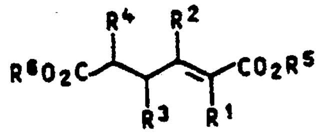

III.

dans lesquelles $R^1$ à $R^4$ ont les significations données ci-dessus et $R^5$ et $R^6$ représentent des atomes d'hydrogène ou sont mis pour des restes alkyle à 1-12 atomes de carbone, pour des restes cycloalkyle à 5 ou 6 atomes de carbone ou pour des restes aralkyle ou aryle, à des températures de 150 à 450° C en présence de catalyseurs solides du type oxyde.

2. Procédé selon la revendication 1, caractérisé en ce qu'on utilise, comme catalyseurs, des oxydes d'éléments des groupes Ia, IIa, IIIa, IVa et Va ou des groupes auxiliaires Ib, IIb, IIIb, IVb, Vb, VIb, VIIb et VIII du système périodique des éléments, des oxydes des métaux des terres rares ou des mélanges de ces oxydes.

3. Procédé selon la revendication 1, caractérisé en ce qu'on utilise de l'oxyde d'aluminium comme catalyseur.

4. Procédé selon l'une quelconque des revendications 1 à 3, caractérisé en ce qu'on conduit la transformation des substances de départ II ou III avec addition d'eau, en choisissant un rapport molaire de 1:0,05 à 1:20 entre la substance de départ de formule II ou III et l'eau.

5. Procédé selon l'une quelconque des revendications 1 à 4, caractérisé en ce qu'on conduit la transformation en lit fluidisé.

6. Procédé selon l'une quelconque des revendications 1 à 6, caractérisé en ce qu'on procède à la transformation à des températures de 300 à 345° C.